**Europäisches Patentamt**

(19) **European Patent Office**

**Office européen des brevets**

(11) Veröffentlichungsnummer: **0 079 491**
**B1**

# (12) EUROPÄISCHE PATENTSCHRIFT

(45) Veröffentlichungstag der Patentschrift:
**10.07.85**

(21) Anmeldenummer: **82109859.7**

(22) Anmeldetag: **26.10.82**

(51) Int. Cl.⁴: **C 07 C 57/05**, C 07 C 51/377,
B 01 J 27/18

(54) Verfahren zur Herstellung von Methacrylsäure.

(30) Priorität: **13.11.81 DE 3145091**

(43) Veröffentlichungstag der Anmeldung:
**25.05.83 Patentblatt 83/21**

(45) Bekanntmachung des Hinweises auf die Patenterteilung:
**10.07.85 Patentblatt 85/28**

(84) Benannte Vertragsstaaten:
**DE FR GB IT NL**

(56) Entgegenhaltungen:
**EP - A - 0 011 225**
**EP - A - 0 024 954**
**EP - A - 0 046 840**
**FR - A - 2 362 108**
**US - A - 4 307 247**
**US - A - 4 314 075**

(73) Patentinhaber: **Röhm GmbH, Kirschenallee**
**Postfach 4242, D-6100 Darmstadt 1 (DE)**

(72) Erfinder: **Gruber, Wilhelm, Dr.,**
**Peter-Behrens-Strasse 34, D-6100 Darmstadt (DE)**

## Beschreibung

Die Erfindung betrifft ein Verfahren gemäß dem Oberbegriff des Patentanspruchs. Gemäß DE-A 2 633 593 werden in einem Verfahren dieser Art Katalysatoren eingesetzt, die auf einem Träger aus mindestens 70% $SiO_2$ mit einer Wasserabsorptionsfähigkeit von mindestens 60% angeordnet sind. Der inneren Oberfläche der Träger wird keine besondere Bedeutung beigemessen. Die verwendeten Träger haben Oberflächenwerte von 2,8 bis 370 cm/g.

In der DE-A 2 722 375 finden sich bei der Beschreibung eines Verfahrens der beschriebenen Art überhaupt keine Angaben über die innere Oberfläche des Trägers. Das gleiche trifft für die EP-A 13 578 zu. Es ist offensichtlich, daß die Bedeutung der inneren Oberflächen des Katalysatorträgers für den Ablauf der Gasphasen-Oxydehydrierung von Isobuttersäure bisher nicht erkannt worden ist.

Zur Dehydrierung von Äthylbenzol zu Styrol wird in EP-A 11 225 ein Katalysator vorgeschlagen, der auf einen Träger mit einer inneren Oberfläche von 0,01 bis 500 qm/g aufgetragen ist. Der Bereich von 2 bis 200 qm/g ist bevorzugt. Bei der Dehydrierung von ungesättigten Aldehyden zu ungesättigten Carbonsäuren, beispielsweise bei der Überführung von Methacrolein in Methacrylsäure, werden nach EP-A 24 954 Molybdän, Vanadin, Phosphor und Sauerstoff enthaltende Katalysatoren auf einem Träger mit einer inneren Oberfläche nicht über 2 qm/g eingesetzt. Für die Anwendung der bekannten Katalysatoren zur Dehydrierung von Isobuttersäure zu Methacrylsäure bestand kein Anlaß.

In den nicht vorveröffentlichten EP-A 46 840, 47 578 und 47 581 sind Katalysatoren mit einer inneren Oberfläche unter 0,5 qm/g nicht offenbart. Für die Dehydrierung von Isobuttersäure zu Methacrylsäure werden nach diesen Druckschriften Katalysatoren auf Trägern mit 1 qm/g auf Kieselgur eingesetzt.

Aus der EP-A 15 569 sind Schalenkatalysatoren für Gasphasen-Oxidationsprozesse bekannt, die auf der äußeren Oberfläche eines Trägers eine 10 bis 1500 µm dicke Schale aus aktiver Katalysatormasse tragen. Als Träger für Katalysatoren, die bei Temperaturen von 350° C oder darüber eingesetzt werden, wird ein unporöses Material oder ein poröses Material mit einem Porendurchmesser von mindestens 20 µm eingesetzt. In jedem Fall darf das Porenvolumen 10 Vol.-% und die innere Oberfläche 5 qm/g nicht überschreiten. Die innere Oberfläche liegt in manchen Fällen unter 0,1 qm/g. Auf diese Träger wird das aktive Material in Form einer wäßrigen Suspension in der Weise aufgebracht, daß es als feste äußere Schale auf dem Träger liegt und gegebenenfalls in den Poren verankert ist. Die Verwendung derartiger Katalysatoren zur Dehydrierung von Isobuttersäure ist nicht bekannt.

Ziel der Erfindung war es, die Ausbeute und Selektivität bei der oxydativen Dehydrierung von Isobuttersäure durch den Einsatz verbesserter Katalysatoren zu steigern. Dieses Ziel wird erfindungsgemäß durch den Einsatz der Katalysatoren gemäß Hauptanspruch erreicht.

Die erfindungsgemäß eingesetzten Katalysatoren enthalten ein Trägermaterial, das zwar ein hohes Porenvolumen, aber eine geringe innere Oberfläche besitzt. Diese Oberfläche befindet sich überwiegend in weiträumigen Poren im Innern des Trägermaterials. Im Gegensatz zur Auftragung des aktiven Materials in Form einer wäßrigen Suspension bei der Herstellung der Katalysatoren gemäß EP-A 15 569, wo die Poren im Trägermaterial nur zur mechanischen Verankerung einer dicken Katalysatorkruste dienen, wird im vorliegenden Fall das aktive Material in Form einer homogenen Lösung in die Poren eingebracht. Die Poren werden im wesentlichen mit aktivem Katalysatormaterial ausgefüllt. Das Volumen des eingebrachten aktiven Materials soll daher nach dem Trocknen und Calcinieren nicht größer als das Porenvolumen sein.

Die Wirksamkeit von Katalysatoren in Gasphasen-Oxidationsverfahren hängt von einer Fülle von Faktoren ab. Dazu zählen neben den oben diskutierten Unterschieden in der Anordnung des aktiven Materials auch dessen Zusammensetzung und Mengenverhältnis zum Träger, weiterhin die Anordnung des Katalysators im Reaktor, die Zusammensetzung des umzusetzenden Gasgemisches, die Umsetzungsbedingungen und andere. Wegen dieser vielfachen Abhängigkeiten wird mit einem bestimmten Katalysatortyp stets eine erhebliche Bandbreite von Ergebniswerten erreicht. Das gilt für Ausbeute-, Selektivitäts- und Raum-Zeit-Ausbeutewerte in gleicher Weise. Eine Verbesserung des Katalysators setzt nicht notwendig voraus, daß die ganze Bandbreite der damit erreichbaren Ergebnisse über den Spitzenergebnissen mit vorbekannten Katalysatoren liegt. Vielmehr liegt eine Verbesserung auch vor, wenn die gesamte Bandbreite auf ein etwas höheres Niveau verlegt wird, das sich mit dem Vergleichsniveau überschneidet, aber zu höheren Spitzenwerten reicht.

Beim Verfahren der DE-A 2 633 593 wird mit einem Katalysator auf einem Trägermaterial mit 20 qm/g Oberfläche ein Spitzenwert der Selektivität von 70% bei 99% Umsatz erreicht. Dieses Ergebnis wird nicht mit solchen Katalysatoren übertroffen, die auf einem Träger mit geringerer Oberfläche aufgebracht sind. Auch mit den in EP-A 13 578 beschriebenen Katalysatoren auf Trägern mit unbekannter Oberflächengröße werden keine über 70% liegenden Selektivitäten für Methacrylsäure erreicht.

Eine Steigerung der Selektivität wird nach DE-A 2 722 375 mit Katalysatoren bewirkt, die aus homogenen Lösungen des aktiven Materials und beispielsweise Diatomeenerde als Träger hergestellt worden sind. Es werden Spitzenwerte über 70% Methacrylsäure-Selektivität, im Höchstfall 75,6% erreicht. Diatomeenerde hat je nach Provenienz und Vorbehandlung Oberflächenwerte zwischen 2,8 und 20 qm/g.

Die Erfindung hat nun eine weitere Steigerung der Methacrylsäure-Selektivität auf einen Spitzenwert über 80% bei einem Umsatz von 97% ermöglicht. Darin kommt eine Verbesserung des Katalysators zum Ausdruck.

## Der Katalysator

### a) Aktive Komponente

Die erfindungsgemäß eingesetzten Katalysatoren enthalten als aktives Material — gegebenenfalls vanadinhaltige — Phosphor-Molybdän-Heteropolysäuren oder ihre Salze. Diese Verbindungen sind orange bis rot gefärbt, verändern aber beim Calcinieren ihre Farbe, so daß anzunehmen ist, daß in dem fertigen Katalysator die Heteropolysäure nicht mehr als solche vorliegt. Wege zur Herstellung dieser Heteropolysäuren sind z. B. in der DE-A 2 722 375 beschrieben. Man erhält sie in Form stark saurer Lösungen durch langdauerndes Kochen von Oxyden oder Oxysäuren des Molybdäns und Vanadins in wäßriger Phosphorsäure. Salze dieser Heteropolysäuren erhält man z. B. dadurch, daß man zusammen mit den oben genannten Molybdän-, Vanadin- und Phosphorverbindungen weitere Metallverbindungen, wie Salze, Oxide oder Hydroxyde, einsetzt.

Den sauren Lösungen der Heteropolysäuren können auch zur Bildung von Salzen als weitere aktive Komponenten Metallsalze zugesetzt werden, die sich vorzugsweise von solchen Säuren ableiten, die sich beim Trocknen und Calcinieren oder spätestens unter den Bedingungen der Oxydehydrierung rückstandslos verflüchtigen. Bevorzugt sind Nitrate und Hydroxyde. Als Beispiele geeigneter Metalle können diejenigen der ersten bis vierten Hauptgruppe (A-Gruppe) und der zweiten bis achten Nebengruppe (B-Gruppe) des periodischen Systems der Elemente genannt werden. Besonders hervorzuheben sind die Metalle K, Mg, Ca, Ba, Al, Ce, La, Pb, Cu, Cr, Fe, No, Ni. Eine getrennte Behandlung des Trägermaterials mit der Heteropolysäure einerseits und dem Metallsalz andererseits ist möglich, aber nicht zweckmäßig. Sie kann jedoch erforderlich werden, falls der Zusatz des Metallsalzes zu der Heteropolysäurelösung zu einer Ausfällung oder Flockung führt, weil sonst die Tränkung des Trägers mit einer homogenen Lösung infragegestellt ist.

Die Atomverhältnisse Mo :V : P können in den zugrundeliegenden Heteropolysäuren im Bereich von (8 bis 12) : (0 bis 3) : 1 liegen. Die bevorzugt eingesetzte Heteropolysäure entspricht der Formel $H_5Mo_{10}V_2PO_{40}$ bzw. $H_2Mo_{10}V_2PO_{38,5}$. Metallsalzzusätze werden vorzugsweise so bemessen, daß ein bis zwei Wasserstoffatome in diesen Formeln durch je ein Metalläquivalent ersetzt werden. Ein Beispiel für eine solche Zusammensetzung ist $CO_{0,5}H_4Mo_{10}V_2PO_{40}$.

### b) Träger

Geeignete Trägermaterialien mit einer Porosität von 10 bis 80% und einer inneren Oberfläche unter 1 qm/g sind an sich bekannt und im Handel erhältlich. Ebenso sind die Bestimmungsmethoden für diese Größen allgemein bekannt. Die Porosität, angegeben in %, ist definiert als

$$\text{Porosität} = \frac{\text{Gewicht des wassergesättigten Trägers} - \text{Gewicht des trockenen Trägers}}{\text{Auftrieb des Trägers in Wasser}} \cdot 100\%$$

Die innere Oberfläche wird nach der BET-Methode bestimmt. Die bevorzugten Werte für das Porenvolumen liegen bei 30 bis 70 Vol.-%. Die innere Oberfläche liegt unter 0,5 qm/g. Die Träger haben häufig die Form von Kugeln, Zylindern, Sattelkörpern oder Granulaten mit Korngrößen von etwa 3 bis 10 mm. Die chemische Natur des Trägermaterials ist nicht kritisch, jedoch muß der Träger eine ausreichende Widerstandsfähigkeit gegen die bei der Katalysatorherstellung und -anwendung auftretenden Belastungen besitzen. Bewährte Träger sind z. B. aus Siliciumdioxid, Aluminiumoxid, Zirkondioxid oder deren Gemischen aufgebaut. Besonders vorteilhaft sind Träger mit einem hohen Gehalt an Siliciumcarbid, gegebenenfalls im Gemisch mit Siliciumdioxid.

### c) Herstellungsverfahren

Der Gehalt an aktivem Material in dem Katalysator soll möglichst hoch sein, aber nicht so hoch, daß das Volumen des aktiven Materials größer als das Porenvolumen ist. Man setzt möglichst konzentrierte Lösungen der aktiven Bestandteile ein. Die Tränkung des Trägermaterials wird mit Vorteil unter Bedingungen vorgenommen, unter denen die in den Poren enthaltene Luft entweichen kann. Bloßes Eintauchen in die wäßrige Lösung reicht häufig nicht aus; zumindest sollte das Tränkgefäß ein- oder mehrmals kurzzeitig evakuiert werden, damit die Luft aus den Poren heraustritt. Man kann auch die Lösung längere Zeit über das Trägermaterial rieseln lassen.

Nach ausreichender Imprägnierung läßt man den nicht aufgenommenen Überschuß der Lösung ablaufen und trocknet anschließend. Das kann an Luft geschehen, wird aber vorzugsweise in einer Trocknungsapparatur, möglichst unter dauernder Bewegung und Luftumwälzung, bei erhöhter Temperatur, z. B. 50 bis 100°C durchgeführt. Anschließend kann, gegebenenfalls unter Vakuum, die Temperatur bis beispielsweise 120°C gesteigert werden. Danach wird bei 250 bis 350°C während 2 bis 10 Stunden calciniert. Nach dieser Behandlung ist der Katalysator einsatzbereit. Der Gehalt an aktivem Material kann durch die Gewichtszunahme nach dem Trocknen oder dem Calcinieren (im Vergleich zu einer Blindprobe) festgestellt werden. Er soll nicht unter 10 Gew.-%, berechnet auf das Gesamtgewicht des fertigen Katalysators, liegen und beträgt vorzugsweise 25 bis 50 Gew.-%.

## Oxydehydrierungsverfahren

Erfindungsgemäß wird der Katalysator zur kontinuierlichen oxydativen Dehydrierung von Isobuttersäure zu Methacrylsäure in der Dampfphase bei Temperaturen oberhalb 300°C, vorzugsweise bei 320 bis 360°C eingesetzt. Das Reaktionsgas enthält pro Mol Isobuttersäure 1 bis 2 Mol Sauerstoff, im allgemeinen in Form von Luft, und 0 bis 3 Mol Wasserdampf. Wenn die Umsetzung wenigstens in der ersten Teilumsetzungsstufe in einem Kreislaufreaktor durchgeführt wird, wobei kontinuierlich ein Teil des aus dem Reaktorraum austretenden Reaktionsgases in den eintretenden Gasstrom zurückgeführt wird, kann auf den Zusatz von Wasserdampf oder von anderen, über den Stickstoffanteil der zur Oxidation eingesetzten Luft hinausgehenden Inertgasen weitgehend oder vollständig verzichtet werden. Zweckmäßigerweise wird an den Kreislaufreaktor ein üblicher Rohrreaktor für die Fortführung des Umsatzes angeschlossen. Wenn die gesamte Umsetzung in einem oder mehreren Rohrreaktoren durchgeführt wird, ist der Zusatz von Inertgasen, wie Wasserdampf, Kohlendioxid oder Stickstoff, im allgemeinen vorteilhaft.

Der Katalysator kann im Dauerbetrieb mit etwa $(1 \text{ bis } 20) \cdot 10^{-2}$ Mol Isobuttersäure je kg Katalysatorfüllung und Minute belastet werden. Man erreicht im Kreislaufreaktor Umsätze von 50 bis 75% der eingesetzten Isobuttersäure, im Rohrreaktor bis nahe an 100%.

## Beispiele

### A) Allgemeine Herstellungsvorschrift für die Katalysatorpräparierung

Durch eine in einem Reaktor aufgeschüttete Schicht von geformten Katalysatorträgern wird eine Lösung der Heteropolysäure oder eines Salzes der Heteropolysäure mit einer Konzentration von 10—80 Gew.-% an katalytisch wirksamen Substanzen kontinuierlich umgepumpt. Die Imprägnierung kann von Raumtemperatur bis ca. 100°C durchgeführt werden. Anschließend werden die so hergestellten Imprägnierungen bei einer Temperatur von etwa 120°C, gegebenenfalls im Vakuum getrocknet. Vor der Durchführung der Oxydehydrierung können die Katalysatoren noch im Temperaturbereich von 250—350°C calciniert werden.

### B) Oxydehydrierung von Isobuttersäure

#### I. Arbeiten im Kreislaufreaktor

Die zu prüfenden Katalysatoren waren in einem Kreislaufreaktor von 7 cm Durchmesser eingebracht. Aus der nachstehenden Tabelle 1 (Beispiele 1 bis 4) gehen die Zusammensetzung und die Form der Katalysatoren sowie Angaben zur inneren Oberfläche der Trägermaterialien, sowie die weiteren Versuchsbedingungen und die erhaltenen Ergebnisse hervor. Die Reaktionstemperaturen betrugen bei den Beispielen 1 bis 4 $340 \pm 10$°C. Eingesetzt wurden immer Isobuttersäure-Sauerstoff (als Luft)-Gemische im Molverhältnis 1 : 1,5.

#### II. Arbeiten im Rohrreaktor

Über Kontaktschüttungen von 58 ml in einem Stahlrohrreaktor mit einem inneren Durchmesser von 2,5 cm wurden dampfförmige Gemische aus Isobuttersäure, Wasser, Sauerstoff und Stickstoff im Molverhältnis 1 : 2 : 1,5 : 20 bei Verweilzeiten von 0,2 bis 0,5 sec und Reaktionstemperaturen von 320 bis 350°C geleitet. Die hierbei erzielten Ergebnisse (Tab. 2, Beispiele 5 bis 7) zeigen auch hier, wie mit dem Vergleichsversuch 8 belegt ist, die deutliche Überlegenheit von Katalysatoren, die mit Trägern von kleiner innerer Oberfläche hergestellt wurden.

Alle Katalysatoren enthielten die Heteropolysäure $H_5Mo_{10}V_2PO_{40}$ als katalytisch aktive Komponente.

### III. Arbeiten in einem Kleinreaktor

In einem Reaktor, der 2,5 g Katalysator mit einer Korngröße von ca. 2 mm enthielt, wurden unter den gleichen Bedingungen wie bei II Katalysatoren mit verschiedenen Trägermaterialien und $H_5Mo_{10}V_2PO_{40}$ als aktiver Komponente bei 330°C Reaktionstemperatur durch Überleiten eines dampfförmigen Isobuttersäure-Wasser-Sauerstoff (als Luft)-Gemisches im Molverhältnis 1 : 2 : 1,7 bei Verweilzeiten von 0,3 sec. getestet. Aus der Tabelle 3 sind die Ergebnisse in den Beispielen 9 bis 11 zu ersehen, wobei die Beispiele 10 und 11 Vergleichsversuche mit Trägern von größerer innerer Oberfläche beschreiben.

Tabelle 1

| Beisp. Nr. | Trägermaterial Form des Katalysatorträgers | chem. Zusammensetzung des Trägers | innere Oberfläche $(m^2/g)$ | Porosität (%) | Zus. d. Heteropolysäure bzw. des Salzes u. deren Menge im Katalysator (Gew.-%) | Katalysatormenge in ml und g | durchges. Isobuttersäure in (Mol/g.min) | Isobuttersäureumsatz (%) | Methacrylsäureselektivität (%) |
|---|---|---|---|---|---|---|---|---|---|
| 1 | Kugel | 65,8% SiC 28,5% $SiO_2$ | 0,01—0,3 | 40—45 | $H_2Mo_{10}V_2PO_{40}$ 25,6 Gew.-% | 145 ml 180 g | 0,00005 | 72 | 75 |
| 2 | Zylinder | 95% $SiO_2$ 4,1% $Al_2O_3$ | 0,1—0,4 | 34—38 | dito 27,6 Gew.% | 250 ml ≙ 330 g | 0,000085 | 68 | 70 |
| 3 | Ring | 94,1% $ZrO_2 + HfO_2$ 3,5% CaO 1,6% $SiO_2$ | 0,1—0,5 | 40—45 | dito 14,7 Gew.-% | 280 ml ≙ 420 g | 0,00002 | 58 | 64 |
| 4 | wie Beispiel Nr. 1 | s. Beisp. Nr. 1 | s. Beisp. Nr. 1 | | $CO_{0,5}H_4Mo_{10}V_2PO_{40}$ 32,7 Gew.-% | 260 ml ≙ 342 g | 0,00003 | 65 | 71 |

0 079 491

Tabelle 2

| Beisp. Nr. | Trägermaterial Form des Katalysatorträgers | chem. Zusammensetzung des Trägers | innere Oberfläche (m²/g) | Porosität (%) | Menge der katalytisch aktiven Substanz im Katalysator (Gew.-%) | Isobuttersäureumsatz (%) | Methacrylsäureselektivität (%) |
|---|---|---|---|---|---|---|---|
| 5 | Kugel | 65,8% SiC<br>28,5% SiO₂ | 0,01—0,3 | 40—45 | 25,6 | 97,1 | 80,2 |
| 6 | Kugel | 99,6% Al₂O₃ | 0,7—1,3 | 54—60 | 35 | 70 | 72 |
| 7 | Kugel | 93,1% Al₂O₃<br>5,6% SiO₂ | 0,3—0,37 | 55—60 | 37 | 78,6 | 75,8 |
| Vergl.-Vers. | | | | | | | |
| 8 | Kugel | 99,85% Al₂O₃ | 220±20 | — | 41 | 63,1 | 31,4 |

Tabelle 3

| Beisp. Nr. | Trägermaterial chem. Zusammensetzung des Trägers | innere Oberfläche (m²/g) | Porosität (%) | Menge der aktiven Komp. im Katalysator (Gew.-%) | Isobuttersäureumsatz (%) | Methacrylsäureselektivität (%) |
|---|---|---|---|---|---|---|
| 9 | SiC | <1 | 10—20 | 17,8 | 20 | 79,8 |
| Vergl.-Vers. | | | | | | |
| 10 | Al₂O₃ | 80 | — | 37,5 | 95,6 | 12,6 |
| 11 | Al₂O₃ | 300 | — | 39,1 | 90 | 16 |

**0 079 491**

**Patentansprüche**

1. Verfahren zur Herstellung von Methacrylsäure durch oxydative Dehydrierung von Isobuttersäure in der Dampfphase bei Temperaturen oberhalb 300°C an einem Katalysator auf Basis der Elemente Molybdän, gegebenenfalls Vanadin, Phosphor und Sauerstoff und gegebenenfalls eines oder mehrerer Metalle als aktive Bestandteile, der auf einem Träger angeordnet ist, dadurch gekennzeichnet, daß ein Katalysator eingesetzt wird, der durch Tränken eines Trägers mit einer Porosität von 10 bis 80% und einer inneren Oberfläche unter 0,5 qm/g mit einer homogenen wäßrigen Lösung der aktiven Katalysatorbestandteile und Trocknen und Calcinieren des getränkten Trägers erhalten worden ist.

2. Verfahren nach Anspruch 1, dadurch gekennzeichnet, daß ein Katalysator eingesetzt wird, der einen Träger mit einem überwiegenden Gehalt an Siliciumcarbid enthält.

3. Verfahren nach den Ansprüchen 1 und 2, dadurch gekennzeichnet, daß ein Katalysator eingesetzt wird, zu dessen Herstellung eine homogene Lösung einer Heteropolysäure der Formel $H_5Mo_{10}V_2PO_{40}$ bzw. $H_2Mo_{10}V_2PO_{38,5}$ oder eines Salzes einer solchen Säure, worin ein bis zwei Wasserstoffatome durch je ein Metalläquivalent ersetzt sind, verwendet worden ist.

**Claims**

1. Process for preparing methacrylic acid by oxidative dehydrogenation of isobutyric acid in the vapour phase at temperatures above 300°C on a catalyst based on the elements molybdenum, optionally vanadium, phosphorus and oxygen and optionally one or more metals as active ingredients, and which is arranged on a carrier, characterised in that a catalyst is used which is obtained by impregnating a carrier with a porosity of from 10 to 80% and an inner surface area of less than 0.5 m²/g with a homogeneous aqueous solution of the active catalyst components and drying and calcining the impregnated carrier.

2. Process as claimed in claim 1, characterised in that a catalyst is used which contains a carrier containing a predominant amount of silicon carbide.

3. Process as claimed in claims 1 and 2, characterised in that a catalyst is used which has been prepared using a homogeneous solution of a heteropoly acid of the formula $H_5Mo_{10}V_2PO_{40}$ or $H_2Mo_{10}V_2PO_{38,5}$ or a salt of such an acid, wherein one to two hydrogen atoms are each replaced by one metal equivalent.

**Revendications**

1. Procédé pour la préparation d'acide méthacrylique par déshydrogénation par voie d'oxydation d'acide isobutyrique en phase gazeuse, à des températures supérieures à 300°C, en présence d'un catalyseur à base des éléments molybdène, éventuellement vanadine, phosphore et oxygène et, le cas échéant, d'un ou de plusieurs métaux en tant que constituant actif qui est appliqué sur un support, caractérisé en ce qu'on utilise un catalyseur qui a été obtenu par imprégnation d'un support ayant une porosité de 10 à 80% et une surface intérieure de moins de 0,5 m²/g, avec une solution aqueuse homogène du constituant actif du catalyseur, puis par séchage et calcination du support imprégné.

2. Procédé selon la revendication 1, caractérisé en ce qu'on utilise un catalyseur qui comprend un support ayant une teneur prépondérante en carbure de silicium.

3. Procédé selon la revendication 1 ou 2, caractérisé en ce qu'on utilise un catalyseur pour la préparation duquel on a utilisé une solution homogène d'un hétéropolyacide de formule $H_5Mo_{10}V_2PO_{40}$ ou $H_2Mo_{10}V_2PO_{38,5}$ ou d'un sel d'un tel acide dans lequel 1 à 2 atomes d'hydrogène sont remplacés chacun par un équivalent métal.